# EUROPEAN PATENT APPLICATION

(11) **EP 3 106 452 A1**
(43) Date of publication of application: **21.12.2016**
(21) Application number: 15172679.1
(22) Date of filing: 18.06.2015
(51) Int. Cl.: C07C 67/00

(54) **PREPARATION OF 2,5,6-TRIHYDROXY-3-HEXENOIC ACID AND 2,5-DIHYDROXY-3-PENTENOIC ACID AND ESTERS THEREOF FROM C6 AND C5 SUGARS**

(71) Applicant: Haldor Topsøe A/S, 2800 Kgs. Lyngby (DK)
(72) Inventor: Taarning, Esben, 2000 Frederiksberg C (DK); Sadaba Zubiri, Irantzu, 2000 Frederiksberg (DK)
(74) Representative: Haldor Topsøe A/S

(57) **Abstract**

Preparation of 2,5,6-trihydroxy-3-hexenoic acid and 2,5-dihydroxy-3-pentenoic acid and esters thereof from C6 and C5 sugars in the presence of a Lewis Acid material, wherein the yield of the 2,5,6-trihydroxy-3-hexenoic acid or 2,5-dihydroxy-3-pentenoic acid or esters thereof is greater than 20 %.

## Description

The present invention relates to the preparation of 2,5,6-trihydroxy-3-hexenoic acid and 2,5-dihydroxy-3-pentenoic acid and esters thereof from C6 and C5 sugars in the presence of a zeolite material.

### Background:

Carbohydrates represent the largest fraction of biomass and various strategies for their efficient use as a feedstock for the preparation of commercial chemicals are being established. Biomass is of particular interest due to its potential for supplementing, and ultimately replacing, petroleum as a feedstock for such purposes. Carbohydrates obtainable from biomass comprise C6 and C5 sugars and are of particular interest industrially as they are a potential source of highly functionalised short chain carbon compounds. This is of particular importance for highly functionalised short chain carbon compounds that are unavailable commercially, such as 2,5,6-trihydroxy-3-hexenoic acid and 2,5-dihydroxy-3-pentenoic acid and esters thereof.

Currently, 2,5,6-trihydroxy-3-hexenoic acid and 2,5-dihydroxy-3-pentenoic acid may be prepared by alkaline degradation of cellulose: Svensk Papperstidning (1974) 16, p 593-602 and J. Appl. Polymer Sci. (1978) 22, pp 615-623; and mannan: Acta Chem Scan. (1980) 40, pp 9-14. However, the product compositions of these reactions comprise numerous compounds and therefore products obtained are in low yields. Additionally, these methods are not industrially feasible due to the large number of products produced.

It is known that C6 and C5 sugars may be substrates in the preparation of methyl lactate in the presence of Sn-BEA. EP 2 184 270 B1 and Science (2010) 328, pp 602 - 605 report yields of methyl lactate of 64%, 43% and 44% at 160 °C in methanol from sucrose, glucose and fructose, respectively. Numerous by-products from this reaction are observed, the major by-product reported is methyl vinylglycolate (3-11%). It has been suggested that trace and small amounts of compounds similar to saccharinic acids may be produced during the disclosed reaction, including a noticeable amount of highly polar products. It has been postulated that these highly polar products are methyl esters of C6 saccharinic acids. Such C6 saccharinic acids are described in Carbohydrate Res. (1996) 280, pp 47-57. However, this reference is silent with regard to the identity, amount in percentage yield and the number of compounds that are components of the highly polar products.

Green Chem. (2012) 14, pp 702-706 discloses similar reaction conditions to Science (2010) 328, pp 602 - 605, wherein the temperature of the reaction is varied. The combined yields of identified products and unconverted sugars are at least 51%.

ChemSusChem (2015) 8, pp 613-617 discloses an increase in methyl lactate yield (from 20-25 % to 66-71%) obtained from sugars in the presence of a heterogeneous stannosilicate catalyst when an alkali ion is added to the reaction process.

Accordingly it is desirable to provide heterogeneous catalytic processes for the preparation of highly functionalized C6 and C5 compounds. Additionally, it is desirable to provide highly functionalized C6 and C5 compounds in high yields via industrially applicable, direct, selective processes.

### Summary of the Invention

It has been discovered that upon selection of specific reaction conditions, 2,5,6-trihydroxy-3-hexenoic acid and 2,5-dihydroxy-3-pentenoic acid and esters thereof from C6 and C5 sugars may be obtained selectively and in high yields.

The present invention provides a process for the preparation of esters of 2,5,6-trihydroxy-3-hexenoic acid or 2,5-dihydroxy-3-pentenoic acid and esters thereof comprising the steps of contacting a C6 or C5 sugar with a Lewis Acid material, wherein the yield of the 2,5,6-trihydroxy-3-hexenoic acid or 2,5-dihydroxy-3-pentenoic acid and esters thereof is greater than 20 %. Preferably the yield of esters is greater than 20%, 25%, 30%.

Preferably the esters of 2,5,6-trihydroxy-3-hexenoic acid or 2,5-dihydroxy-3-pentenoic acid are methyl esters. 2,5,6-trihydroxy-3-hexenoic acid methyl ester and 2,5-dihydroxy-3-pentenoic acid methyl ester may also be known as 'THM' and 'DPM'.

The yield of the 2,5,6-trihydroxy-3-hexenoic acid or 2,5-dihydroxy-3-pentenoic acid and esters thereof is calculated based on the sugar starting material.

C6 or C5 sugar means sucrose, glucose or xylose and isomers thereof. Isomers of glucose include fructose, galactose and mannose. Isomers of xylose include xylulose and lyxose.

Lewis Acid materials include materials that are homogenous with alcohols and water, such as tin chloride, materials that are heterogenous with alcohols and water, such as solid Lewis Acids.

Solid Lewis Acid materials may be crystalline or non-crystalline. Non-crystalline solid Lewis Acid materials include ordered mesoporous amorphous, such as Sn-MCM-41 and Sn-SBA-15, or other mesoporous amorphous forms. Crystalline microporous material includes zeolite materials and zeotype materials.

Zeolite materials are crystalline alumino-silicates with a microporous crystalline structure, according to Corma et al., Chem. Rev. 1995, 95 pp 559-614.

A zeotype material is where the aluminum atoms of a zeolite material are partly or fully substituted by a metal (metal atoms) such as zirconium (Zr), titanium (Ti) and tin (Sn). Zeolite materials are crystalline alumino-silicates with a microporous crystalline structure, according to Corma et al., Chem. Rev. 1995, 95 pp 559-614.

The present invention relates to a process wherein the zeotype material framework structure is selected from the group consisting of BEA, MFI, FAU, MOR, FER, MWW, MCM-41 and SBA-15.

The present invention relates to a process wherein the zeotype material comprises an active metal selected from one or more of the group consisting of Sn, Ti, Pb, Zr, Ge and Hf.

The present invention relates to a process wherein the zeotype material is selected from the group consisting of Sn-BEA, Sn-MFI, Sn-FAU, Sn-MOR, Sn-MWW, Sn-MCM-41 and Sn-SBA-15. Preferably the zeotype material is Sn-BEA.

In a further embodiment of the invention, the Sn-BEA is prepared by a direct synthesis method (using hydrogen fluoride) or by a post treatment process (method). Examples of direct synthesis methods are described in EP 1 010 667 B1 and Chem Commun. (1997) pp 425-426. The Sn-BEA is prepared by fluoride direct synthesis method or by a post treatment process in order to avoid the presence of alkaline components in the Sn-BEA, Such alkaline components are, for example: potassium ions as illustrated in WO2015/024875 A1. Preferably, any alkaline material present in the reaction solution is present in a concentration of less than 0.13 mM or in an amount of less than 0.5 wt % of the catalyst composition.

An example of a post treatment process for the preparation of Sn-BEA is illustrated WO2015/024875 A1 (Catalyst A). The hydrogen fluoride route (also known as the direct synthesis method), is described in EP 1 010 667 B1 and Chem Commun. (1997) pp 425-426.

In a further aspect of the invention, the process is carried out at a temperature from 110 °C to 200 °C, from 110 °C to 190 °C, from 110 to 180 °C, preferably at a temperature from 140 to 170 °C.

In a further aspect of the invention, the solvent is selected from the group consisting of methanol, ethanol, water and mixtures thereof.

### Examples:

### Preparation of Sn-BEA:

### A. Process for the preparation of Sn-BEA via a direct synthesis method (HF route).

Sn-Beta zeolites were synthesized by modifying the route described by Valencia et al.[US6306364 B1] In a typical synthesis procedure, 30.6 g f tetraethyl orthosilicate (TEOS, Aldrich, 98%) was added to 33.1 g of tetraethylammonium hydroxide (TEAOH, Sigma-Aldrich, 35% in water) under careful stirring, forming a two-phase solution. After stirring (∼60 min) one phase is obtained and tin(IV) chloride pentahydrate (SnCl 4 ·5H₂O, Aldrich, 98%) dissolved in 2.0 mL of demineralized water was added drop wise. Stirring was maintained for several hours to allow ethanol formed from the hydrolysis of TEOS to evaporate. Finally, 3.1 g hydrofluoric acid (HF, Fluka, 47-51%) in 1.6 g of demineralized water was added to the gel, yielding a solid with the molar composition; 1.0Si:0.005Sn:0.02Cl⁻:O.55TEA⁺:0.55F⁻:7.5H₂O. All samples were then homogenized and transferred to a Teflon-container placed in a stainless steel autoclave. This was then placed at 140°C for 14 days. The solid was recovered by filtration and washed with demineralized water, followed by drying overnight at 80°C in air. The organic template contained within the material was removed by heating the sample at 2°C/min to 550°C in static air and maintaining this temperature for 6 h.

### B. Process of preparing Sn-BEA via a post-treatment method.

Sn/Beta (Si/Sn = 125) is prepared according to the procedure described in ChemSusChem 2015, 8, 613-617. Commercial zeolite Beta (Zeolyst, Si/Al 12.5, NH4⁺ form) is calcined (550°C for 6 h) to obtain the H⁺ form and treated with 10 g of concentrated nitric acid (HNO₃, Sigma-Aldrich, ≥65%) per gram of zeolite Beta powder for 12 h at 80°C. The resulting solid is filtered, washed with ample water and calcined (550°C for 6 h using a ramp of 2°C/min) to obtain the dealuminated Beta. This solid is impregnated by incipient wetness methodology with a Si/Sn ratio of 125. For this purpose, tin(II) chloride (0.128 g, Sigma-Aldrich, 98%) is dissolved in water (5.75 mL) and added to the dealuminated Beta (5 g). After the impregnation process, the samples are dried 12 h at 110°C and calcined again (550°C for 6 h).

### Example 1:

In a typical reaction, 0.150 g of alkali-free Sn-Beta zeolite (Si/Sn = 150), 1.31 mmol of glucose and 15.0 g of anhydrous methanol (15.0 g, Sigma-Aldrich, >99.8%) is added to a stainless steel pressure vessel (40 cc, Swagelok). The reactor is closed and placed in a preheated oil bath at 100°C under stirring (700 rpm) and allowed to react for 20 hours. After reaction the vessel is rapidly cooled by submerging the reactor in cold water. The sugar derivative was identified by GC-MS (Agilent 6890 with a Phenomenex Zebron ZB-5 column equipped with an Agilent 5973 mass selective detector).

### Examples with high yield

Examples 2 - 3: Example 1 was followed where the temperature of the process was increased to 160 °C and 170 °C. Catalyst used is Sn-Beta (Si/Sn = 150) according to method A.

**Table 1: Yield of 2,5,6-trihydroxy-3-hexenoic acid methyl ester (THM) from a C6 sugar (glucose) at varying process temperatures.**

| Example | Temperature (°C) | Yield (%) |
|---|---|---|
| 1 | 140 | 17.6 |
| 2 | 160 | 14.5 |
| 3 | 170 | 17.3 |

Examples 4 - 6: Example 1 was followed where the starting material was xylose (instead of glucose) at 160 °C and different catalysts were used.

**Table 2: Yield of 2,5-dihydroxy-3-pentenoic acid methyl ester (DPM) from a C5 sugar (xylose) with different catalysts.**

| Example | Catalyst | Yield (%) |
|---|---|---|
| 4 | Method A (Si/Sn = 200) | 27.5 |
| 5 | Method A (Si/Sn = 150) | 24.5 |
| 6 | Method B (Si/Sn = 125) | 18.1 |

## Claims

1. A process for the preparation of 2,5,6-trihydroxy-3-hexenoic acid or 2,5-dihydroxy-3-pentenoic acid or esters thereof comprising the steps of contacting one or more C6 or C5 sugars with a Lewis Acid material, wherein the yield of the 2,5,6-trihydroxy-3-hexenoic acid or 2,5-dihydroxy-3-pentenoic acid or esters thereof is greater than 20 %.

2. A process according to claim 1, wherein the esters of 2,5,6-trihydroxy-3-hexenoic acid or 2,5-dihydroxy-3-pentenoic acid are 2,5,6-trihydroxy-3-hexenoic acid methyl ester and 2,5-dihydroxy-3-pentenoic acid methyl ester.

3. A process according to claims 1 and 2, wherein the one or more C6 or C5 sugars is selected from one or more of the group consisting of sucrose, galactose, glucose and fructose.

4. A process according to claims 1 to 3, wherein the temperature of the process is from 110 to 190 °C.

5. A process according to claims 1 to 3, wherein the temperature of the process is from 140 to 170 °C.

6. A process according to claims 1 to 5, wherein the Lewis Acid material is Sn-BEA.

7. A process according to claim 6, wherein the catalyst is Sn-BEA prepared by a direct synthesis or a post-treatment method.

8. A process according to claims 1 to 7, wherein any alkaline material present in the reaction solution is present in a concentration of less than 0.13 mM or an amount of less than 0.5 wt % of the catalyst composition.
